# EUROPEAN PATENT SPECIFICATION

(11) **EP 0 433 378 B1**
(45) Date of publication and mention of the grant of the patent: **25.10.1995**
(21) Application number: 89910586.0
(22) Date of filing: 12.09.1989
(51) Int. Cl.: C07K 7/06, C07K 14/445, C12N 15/00

(54) **ALLELIC VARIANTS OF $i(PLASMODIUM FALCIPARUM) MEROZOITE SURFACE ANTIGEN**
ALLELISCHE VARIANTEN VON -I(PLASMODIUM FALCIPARUM) MEROZOIT-OBERFLÄCHEN-ANTIGEN
VARIANTES ALLELIQUES D'UN ANTIGENE DE SURFACE DE MEROZOITE DU $i(PLASMODIUM FALCIPARUM)

(30) Priority: 12.09.1988 AU 382/88; 24.08.1989 AU 5962/89
(43) Date of publication of application: 26.06.1991
(73) Proprietor: SARAMANE PTY. LTD., Kooyong, VIC 3144 (AU)
(72) Inventor: SMYTHE, Jason, Arthur, Edithvale, VIC 3196 (AU); ANDERS, Robin, Frederic, North Melbourne, VIC 3051 (AU); KEMP, David, James, North Balwyn, VIC 3103 (AU); COPPEL, Ross, Leon, Armadale, VIC 3143 (AU); SAUL, Allan, James, The Gap, QLD 4061 (AU); JONES, Graham, Lloyd, Rainworth, QLD 4065 (AU); IRVING, David, Owen, Lane Cove, NSW 2066 (AU); DYER, Sandra, Leigh, Chatswood, NSW 2067 (AU); CHEE, David, Wai, Wing, East Killara, NSW 2071 (AU); GOSS, Neil, Howard, Wahroonga, NSW 2076 (AU)
(74) Representative: Dzieglewska, Hanna Eva
(86) International application number: AU8900388
(87) International publication number: WO9002752

(56) References cited:
- AU-A- 7 699 887
- US-A- 4 767 622
- US-A- 4 835 529
- No further relevant documents have been disclosed.
- Molecular and Biochemical Parasitology, Vol. 28 No. 1, 1988 (Elsevier N.Y.)EPPING et al. "An Epitope Recognised by Inhibitory Monoclonal Antibody thatReacts with a 51 Kilodalton Merozoite Surface Antigen in PlasmodiumFalciparum", see pages 1-10 especially page 1, column 2, page 6, column 2 -page 7
- Molecular and Biochemical Parasitology, Vol. 32, No. 1 1989 (Elsvier N.Y.)CLARK et al., "46-53 Kilodalton Glycoprotein from the Surface of PlasmodiumFalciparum Merozoites", see pages 15-24 especially column 2 - page 23.

## Description

This invention relates to the identification of antigens of the asexual blood stages of Plasmodium falciparum which are capable of generating antibodies which are able to inhibit the growth of the parasite, and to the use of these antigens and antibodies in immunization and treatment methods.

As is described in prior International Patent Specification No. PCT/AU87/00227, merozoite surface antigens are of particular interest in the study of malarial immunology and the mechanism of host protection to parasites due to their accessibility to host immune mechanisms and because of their likely involvement in the erythrocytic invasion process. Immunity against erythrocytic stages of Plasmodium infections has been demonstrated by simian vaccination studies using merozoite preparations of P.knowlesi(1) and P.falciparum(2,3). Antigens identified by ¹²⁵I-surface labelling studies (4,5) are likely to reside on the merozoite surface membrane whereas the localization of those antigens which are immunoprecipitated with immune serum (6,8) is more tenuous. One high Mᵣ antigen of Plasmodium falciparum (9) which is synthesized predominantly in schizonts and processed to generate proteins of Mᵣ 88, 42 and 19 kDa (10,11) upon schizont maturation is the precursor to the major merozoite surface antigen (PMMSA, also called MSA1). This group of antigens provide a degree of immunity to malaria in Saimiri monkeys(12). They have been described in several Plasmodium falciparum strains, (9,13-15) and Plasmodium species(16,17).

A range of other malarial antigens have also been described in the literature. These include for example a 51kd merozoite surface antigen of P. falciparum described by Epping et al. in Mol. Biochem. Parasitol., 28(1) p1-10, 1988. Clark et al. in Mol. Biochem. Parasitol., 32(1) p15-25, 1989, describe a 46-53kd glycoprotein from the surface of P.falciparum merozoites. US-A-4,825,259 describes a range of antigenic glycoproteins present on the surface of P.falciparum merozoites, including glycoproteins of molecular weights of about 56 and about 50kd. Soluble proteinaceous antigens isolated from the culture fluid of in vitro propagated plasmodium sp. parasites in mammalian erthyrocyte culture supernatant or from washes of cultured parasitized erythrocytes, are described in WO85/00976.

International Patent Specification No. PCT/AU87/00227 describes a second merozoite surface antigen of the asexual blood stages of Plasmodium falciparum which is characterised by
(i) having a relative molecular mass, Mᵣ, (determined by SDS-polyacrylamide gel electrophoresis) in the range of approximately 41,000 to 53,000;
(ii) being a glycoprotein incorporating myristic acid;
(iii) being present firstly as a diffuse cytoplasmic localisation and in mature schizonts being located on the surface membrane of merozoites; and
(iv) being recognised by some monoclonal antibodies against the asexual blood stages of P.falciparum that inhibit parasite growth in vitro.

This second merozoite surface antigen has been given the names GYMSSA, QF122, Mᵣ 45,000 MSA and MSA2. The latter name will be used throughout the present specification to refer to this second merozoite surface antigen. A clone (Ag 513) expressing this antigen has been isolated from a library of λ-Amp3 clones expressing P.falciparum cDNA sequences. The nucleotide sequence Ag513, together with the predicted amino acid sequence is set out in the earlier patent specification, together with experimental detail indicating that the size and the physical characteristics of the antigen encoded by Ag513 are the same as that of QF122, results which were confirmed by colony immunoassays in which monoclonal antibodies to QF122 reacted strongly with Ag513 but not other P.falciparum antigen-expressing cDNA clones.

The larger merozoite surface antigen, PMMSA or MSA1, has been shown to exhibit extensive antigenic diversity with the corresponding proteins from different isolates consisting of a series of conserved and variable domains. Similarly, it has now been shown that the smaller merozoite surface antigen, MSA2, also shows marked antigenic diversity.

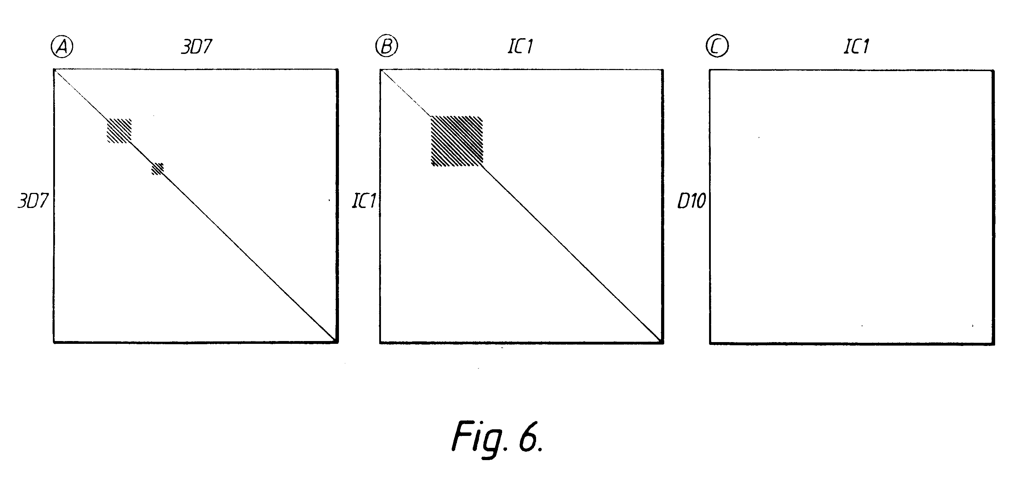

The nucleotide sequence and predicted amino acid sequence of Ag513 are shown in Figure 6 of International Patent Specification No. PCT/AU87/00227, the sequence depicted there being derived from the P.falciparum isolate FCQ27/PNG (FC27). Further sequencing work which has now been done on other isolates of P.falciparum has revealed allelic variation of the MSA2, with the sequences derived from various isolates having high homology in regions of 43 amino acids at the N-terminal end and 73 amino acids at the C-terminal end. This work has established that there are at least two "families" of allelic variants of MSA2, and that certain peptides from the conserved regions which are common to all variants are immunogenic and therefore may form the basis of a synthetic peptide vaccine.

In any synthetic vaccine, the antibody to the immunogen must both recognise the authentic molecule and possess the necessary biological properties to confer protection. Malaria has proved a useful system for the study of peptide based vaccines. Antibodies raised to various sized synthetic repeats of the circumsporozoite protein 4 amino acid subunit (NANP), reacted efficiently with the native protein in several assay systems, inhibited the interaction of sporozoites with their target cells in vitro and neutralized sporozoite infectivity(19). Similarly, antibodies raised against the C-terminal repeat of the blood stage antigen RESA/Pf155 (EENVEHDA) have been shown to react with the native protein and to inhibit efficiently parasite invasion in vitro(20). Recently, Pattarroyo et.al.(21) have produced a carrier free synthetic peptide based vaccine which appears to protect partially humans against challenge with asexual stages of P.falciparum. These workers used peptides that had been previously demonstrated to induce partial protection in A. triviratus monkeys. Richman and Reese(22) have also shown that a carrier free peptide from the P.falciparum heat shock protein was capable of raising antibodies reactive against the native protein.

According to the present invention, there is provided a class of merozoite surface antigens of P.falciparum which are characterised by:
(i) having a relative molecular mass, Mᵣ, (determined by SDS-polyacrylamide gel electrophoresis) in the range of approximately 41,000 to 53,000; and
(ii) comprising multiple allelic forms of MSA2, having conserved N-terminal and C-terminal sequences.

In particular, the antigens of the present invention exhibit high homology (greater than 90% homology) in the N-terminal sequence of 43 amino acids and the C-terminal sequence of 74 amino acids, the N-terminal sequence including the signal sequence with a hydrophobic core of approximately 15 residues and the C-terminal sequence including a second very hydrophobic sequence, approximately 17 residues in length and presumed to be a signal for attachment of a glycosylphosphatidyl inositol membrane anchor. A further feature of the antigens of the present invention is the presence of centrally located repetitive sequences that may be diverse in length, number and sequence.

The present invention also encompasses a vaccine composition comprising at least two allelic forms of MSA2 as broadly described above, or antigenic fragments thereof, together with a pharmaceutically acceptable carrier or diluent, and optionally an adjuvant, as well as a method for actively immunizing a host against P.falciparum, which method comprises administering to the host a vaccine composition as broadly described above.

Antigenic fragments of particular interest in accordance with the present invention are fragments from the conserved N-terminal and C-terminal regions. In particular, certain peptides from these regions have been found to elicit antibodies which react appropriately on immunofluorescence or immunoblotting analysis with intact MSA2 from different strains of P.falciparum. In this aspect, the present invention provides synthetic peptides capable of eliciting antibodies which recognise intact MSA2, which are characterised in that they consist of or contain an amino acid sequence selected from the group consisting of:
(i) SNTFINNA (E71);
(ii) QHGMHGS (G5); and
(iii) NTSDSQKE (G12);
or antigenically active portions thereof.

The present invention also extends to a vaccine composition for eliciting the production of antibodies which recognise intact MSA2, which is characterised in that it comprises at least one synthetic peptide as broadly described above. The vaccine composition may further comprise a pharmaceutically acceptable carrier or diluent, and optionally an adjuvant. The invention also extends to a method of actively immunising a host by eliciting the production of antibodies which recognise intact MSA2, which method comprises the administration to the host of a vaccine composition as described above.

In a further aspect of the present invention, there are provided synthetic peptides capable of eliciting specific antibodies against the FC27, Indochina 1 or other variants of MSA2, which are characterised in that they consist of or contain an amino acid sequence corresponding to one of the sequence repeats in MSA2, or are selected from the group consisting of:
(iv) MANEGSNT (E87);
(v) ANEGSNTN (E88);
(vi) NEGSNTNS (E89); and
(vii) SSENPNHN (G34)
or antigenically active portions thereof.

These synthetic peptides eliciting specific antibody responses against specific variants of MSA2 may be used alone or in combination with any of the other synthetic peptides described herein in a vaccine composition to generate an immune response against different variants of MSA2.

The synthetic peptides of the present invention may be prepared by any suitable method, for example, by chemical synthesis or by expression of an appropriate nucleotide sequence in an appropriate host cell using the well known recombinant DNA techniques.

In order to enhance the immunogenicity of the synthetic peptides of this invention, they may be coupled or conjugated to a suitable carrier protein, such as diphtheria toxoid (DT).

In general terms, a vaccine composition in accordance with the present invention may be a multicomponent vaccine comprising antigens as broadly described herein from different families of MSA2 allelic variants or antigenic fragments thereof. Alternatively, the vaccine composition may comprise one or more antigenic peptides corresponding to the conserved region or fragments thereof, or the variable regions representative of different MSA2 allelic variants.

The present invention also encompasses a recombinant DNA molecule comprising all or a portion of a nucleotide sequence which is capable of being expressed as a polypeptide having the antigenicity of one or more allelic forms of MSA2 as broadly described above, or antigenic fragments thereof, or a recombinant cloning vehicle or vector or a host cell comprising a said recombinant DNA molecule. Suitable host cells include, for example, bacteria and yeast, however the present invention also extends to expression in mammalian or insect cell lines using for example recombinant vaccinia virus or recombinant baculovirus. In this aspect, the present invention also extends to a synthetic polypeptide prepared by expression of all or a portion of a nucleotide sequence comprised in a said DNA recombinant molecule, together with a vaccine composition comprising at least one such synthetic polypeptide.

In work leading to the present invention, complete nucleotide sequences of allelic forms of MSA2 have been obtained from five isolates of P.falciparum (FC27, Indochina 1, 3D7, MAD71 and K1). In addition, the MSA2 of two different isolates has been expressed in E.coli both as a fusion protein and as a non-fused protein, and the immunogenicity of the expressed proteins has been demonstrated.

Further features of the present invention will be apparent from the detailed description in the following Examples.

### EXAMPLE 1

DNA corresponding to the MSA2 gene in the Indochina 1, 3D7, MAD71 and K1 isolates was amplified by the method of polymerase chain reaction (PCR) using DNA primers derived from the first and last 30 nucleotides of the Ag513 (FC27 clone) sequence as shown in Figure 6 of International Patent Specification No. PCT/AU87/00227. The amplified DNA was then subjected to sequence analysis by known methods.

The polymerase chain reaction (PCR) procedure was based on that described by Saiki et.al.(24), using Taq polymerase isolated from Thermus aquaticus (Perkin Elmer Cetus). Oligonucleotides used as PCR primers corresponded to the first and last 30 nucleotides of the translated FC27 MSA2 gene sequence (nucleotides 94-123 and 859-888) and the gene was amplified through 25 PCR cycles. The reaction mixture contained 100 ng of total genomic parasite DNA, 1µM of each primer, 200µM of each dNTP, in a final vol. of 100 µl of 50 mM KCL, 10 mM Tris. Cl (pH 8.3), 1.5 mM MgCl₂, 0.01% (W/V) gelatin. The PCR products were gel purified, ligated into the appropriate M13 vectors, and sequenced by the chain termination method as described(25).

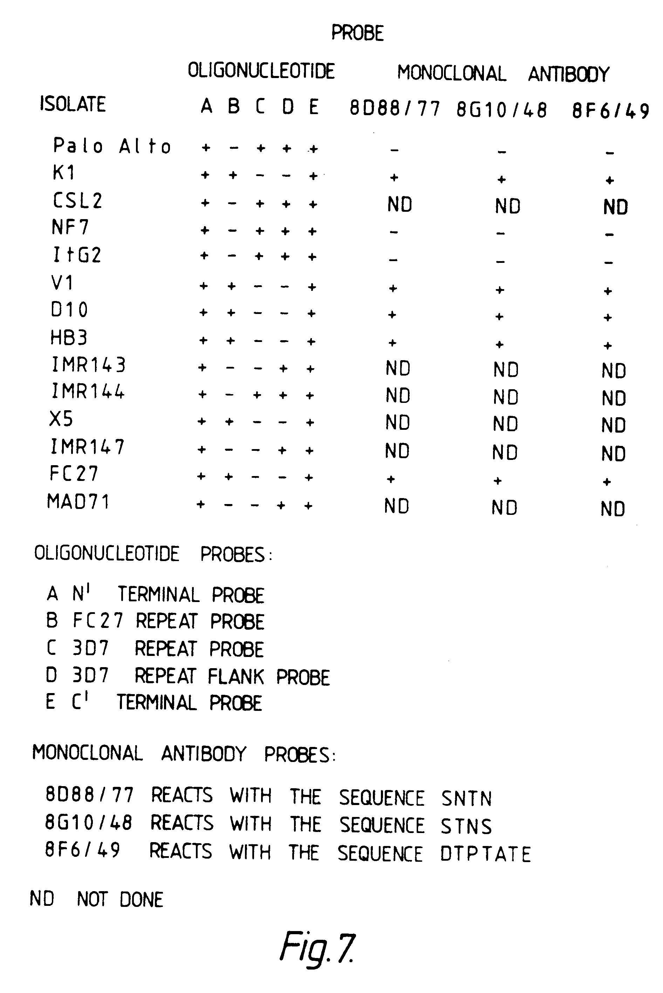

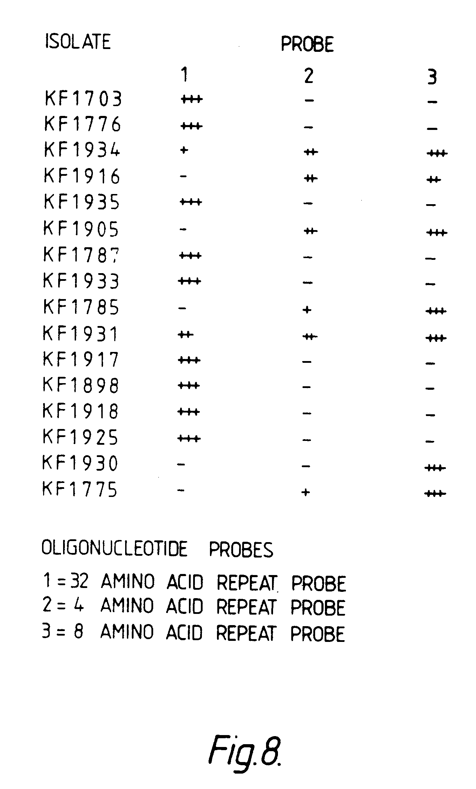

Samples of DNA amplified by the PCR were also dot-blotted onto nitrocellulose and hybridized with oligonucleotide probes as previously described(26). The 5' dephosphorylated oligonucleotide (50 pM) probes were end-labelled by incubation with polynucleotide kinase (Boehringer Mannheim) in the presence of 150 µCi [γ-³²P] ATP (>5000 Ci mmol⁻¹, Amersham). Probes used in the dot-blot hybridization of Figure 7 hereinafter, were: Probe A, 5' conserved region probe corresponding to the first 30 base pairs (bp) of the translated FC27 MSA 2 allele, including the initiation codon. Probe B, FC27 repeat-specific probe corresponding to the first 21 bp of the 96 bp repeat of the FC27 MSA2 allele. Probe C, 3D7/Indochina 1 repeat-specific probe (GGTGGTAGTGCTGGTGGTAG) from the MSA2 gene of 3D7 and Indochina 1. Probe D, 3D7 and Indochina 1 repeat-flanking probe corresponding to a 20 bp sequence (GCATCTTGAGTGGGTGGAAC) that is found in the 3D7 and Indochina 1 MSA2 genes but not in the FC27 MSA2 gene. Probe E, 3' conserved region probe corresponding to the last 30 bp (including the termination codon) of the FC27 MSA2 allele. Probes used in the hybridization of Figure 8 hereinafter were: Probe 1, repeat-specific probe (ATCACAAACTACTACTC) from the MSA2 gene of FC27. Probe 2, repeat-specific probe (GGTGGTAGTGCTGGTGGTAG) from the MSA2 gene of 3D7/Indochina 1. Probe 3, repeat-specific probe (CAGAACCAGCAACA) from the MSA2 gene of MAD71.

The results are shown in the accompanying drawings.

In the drawings:

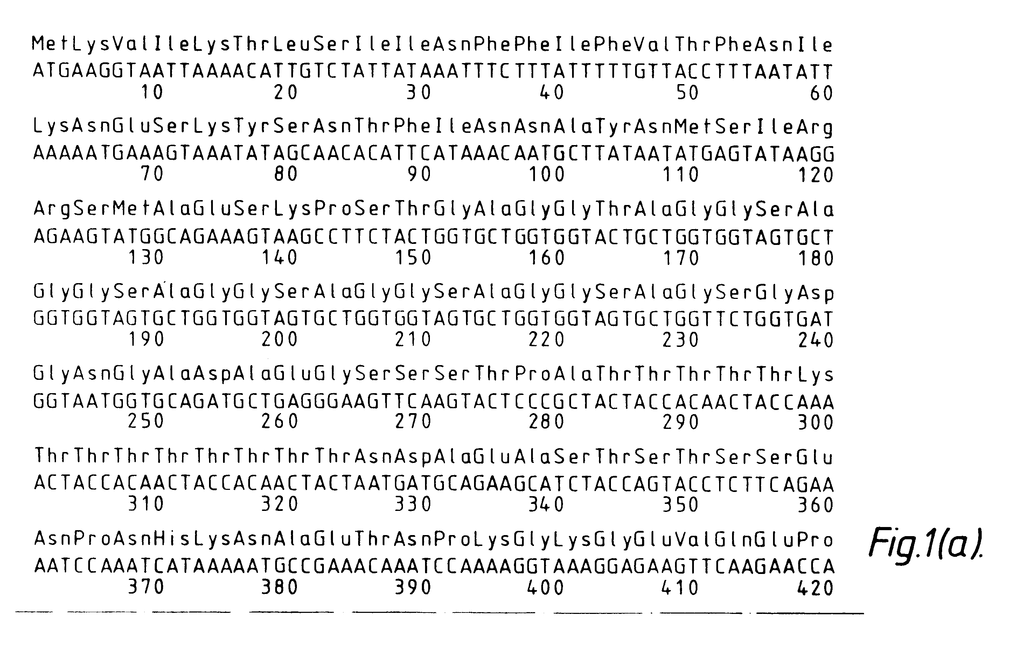

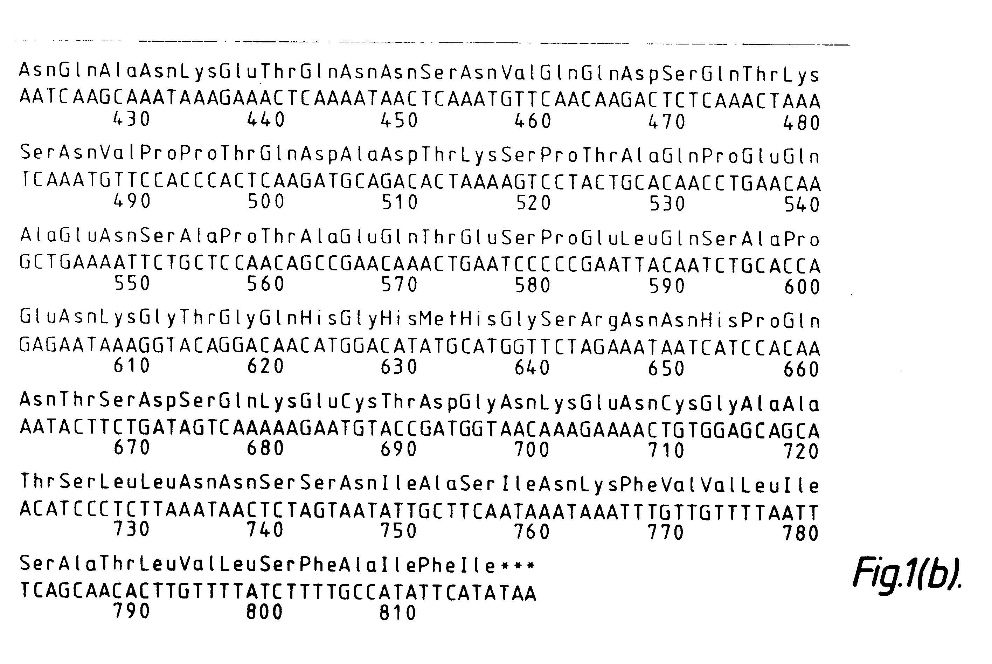

Figure 1 shows the nucleotide sequence and the translated polypeptide for MSA2 of the 3D7 cloned line of P.falciparum, from initiation codon to termination codon. As depicted, there are two repeat sequences present, a GGSA repeat represented 6 times, and a threonine repeat sequence commencing at base 283.
Figure 2 is the complete nucleotide sequence and the translated polypeptides of the Indochina 1 isolate of P.falciparum, in which the number of GGSA repeats is expanded to 12 copies, and which contains a smaller number of threonine repeats.
Figure 3 is the complete nucleotide sequence and the translated polypeptide for MSA2 of the MAD71 isolate of P.falciparum, which contains a AGAVAGSG repeat sequence represented 6 times.

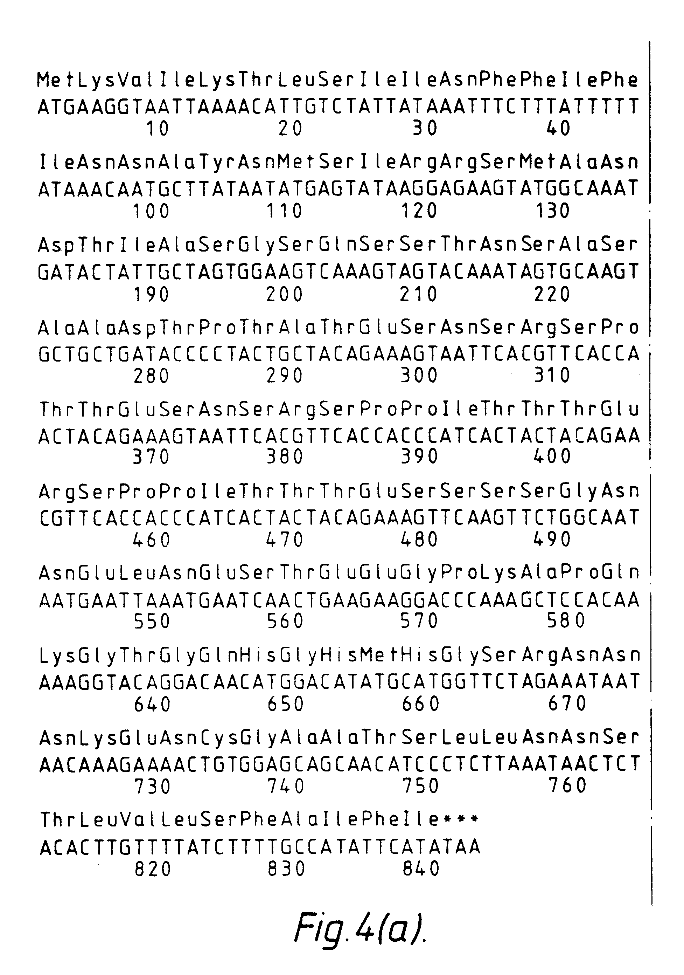

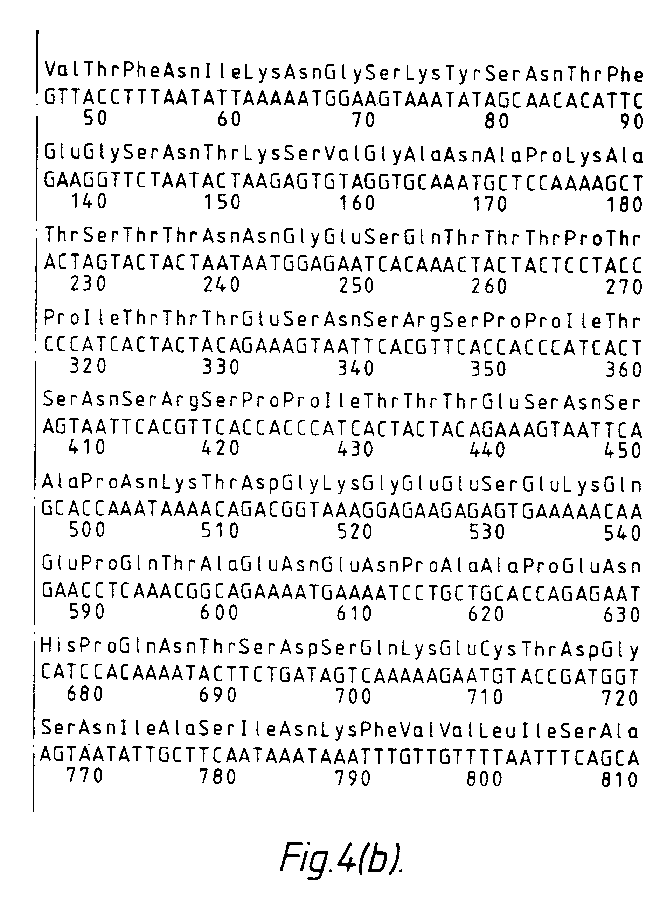

Figure 4 is the complete nucleotide sequence and the translated polypeptide for MSA2 of the K1 isolate of P.falciparum, which contains one copy of the 32 amino acid repeat found in MSA2 of isolate FC27 (see Figure 6 of International Patent Specification No. PCT/AU87/00227), and a TTTESNSRSPPI repeat represented 4 times.

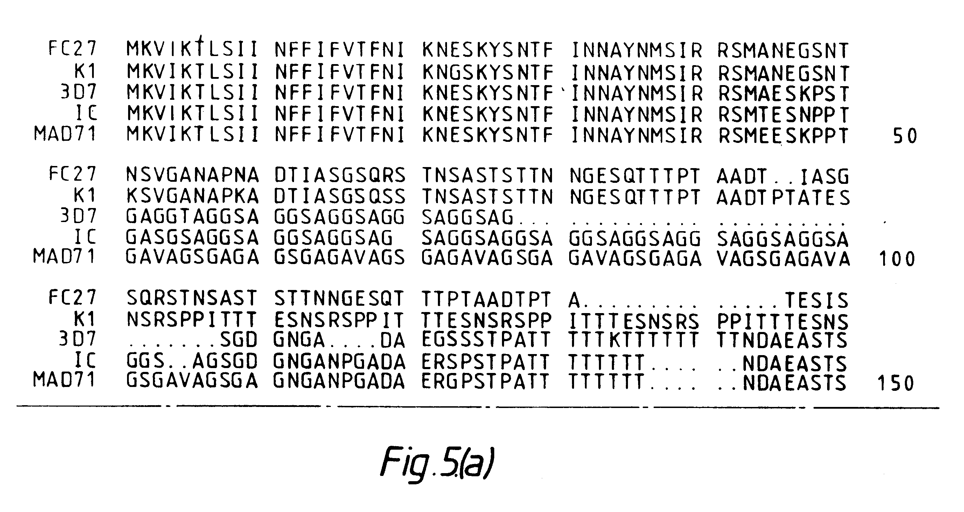

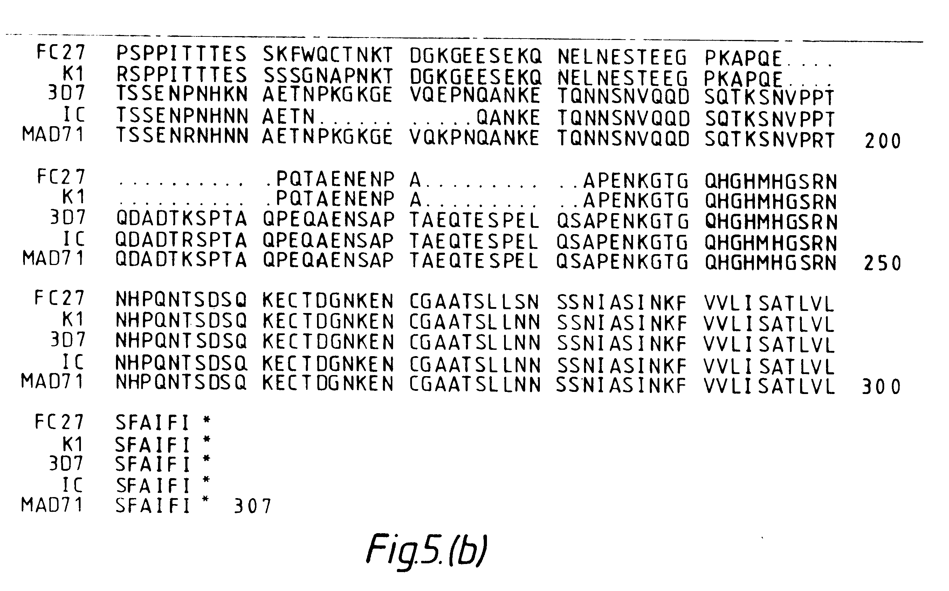

Figure 5 is a comparison of the deduced amino acid sequences for the different MSA2 polypeptides.
Figure 6 shows three computer generated "diagon" nucleotide sequence comparisons:
   (a) a comparison of the 3D7 sequence with itself, showing an upper block of the GGSA repeat and a lower block of the threonine repeat.
   (b) a comparison of the Indochina 1 sequence against itself, demonstrating an expansion in the GGSA repeat, and a reduction in the secondary repeat block of the threonine residues; and
   (c) compares the Indochina 1 and the FC27 sequences, showing that these sequences have conserved regions of 43 amino acids and 74 amino acids at the 5' and 3' ends respectively.
Figure 7 is a summary of dot blots in which oligonucleotide probes based on the conserved N and C-terminal sequences, the FC27 repeat sequence, the 3D7 repeat sequence and a 3D7 repeat flank sequence were hybridized to DNA (amplified by polymerase chain reaction) from 14 different isolates of P.falciparum which had been in long term culture in vitro. These results demonstrate that there are multiple allelic forms of the MSA2. The figure also shows reaction of some of these isolates with monoclonal antibodies 8D88/77, 8G10/48 and 8F6/49 (see International Patent Specification No. PCT/AU87/00227). These antibodies only react with isolates that hybridize with the probe for the FC27 repeat sequence.
Figure 8 is a summary of dot blots in which oligonucleotide probes based on the FC27, 3D7 and MAD71 repeat sequences were hybridized to DNA amplified by PCR from 16 isolates of P.falciparum recently established in culture in vitro. These results again show that there are different allelic forms of MSA2, and that all isolates react with at least one of the three different repeat probes. Some DNA preparations reacted with more than one probe; this may represent mixed infections (for example KF 1931 or KF 1934), or MSA2 sequences intermediate between the 3D7 and MAD71 repeat types (for example KF 1905).

### EXAMPLE 2

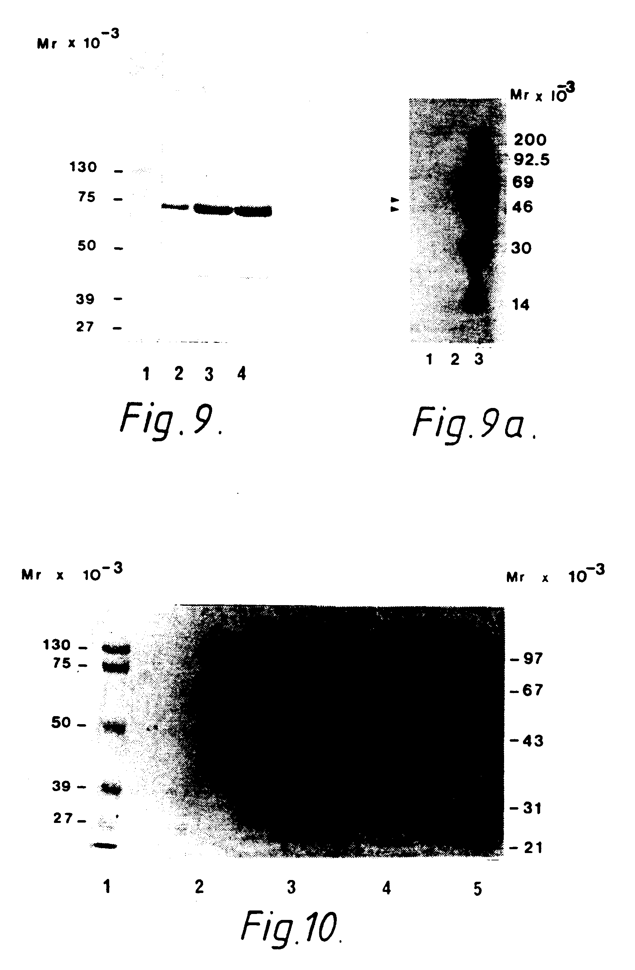

The MSA2 of the Indochina 1 isolate of P.falciparum has been expressed in E.coli as a fusion protein with the glutathione S-transferase (GST) of Schistosoma japonicum. The expression construct was prepared by ligating the SspI fragment of the MSA2 gene(27) into the SmaI site of the expression vector, pGEX-3X(28). In so doing, the amino terminal signal sequence and the carboxy terminal hydrophobic domain are excluded from the recombinant molecule. The fusion protein was localised in the soluble fraction of IPTG-induced E.coli cells and was isolated away from bacterial proteins using affinity chromatography(28). The purified protein consisted of a major species of Mᵣ∼70,000 along with minor components of Mᵣ∼45,000 and Mᵣ∼30,000 as shown in Fig.9, which depicts a Coomassie blue stained SDS polyacrylamide gel of the purified MSA2 fusion protein (Lane 1, Molecular weight markers; Lane 2, 0.5µg MSA2 fusion protein; Lane 3, 2µg MSA2 fusion protein; Lane 4, 4µg MSA2 fusion protein).

The Mᵣ∼70,000 component, which was estimated to be approximately 90% of the total purified protein, reacted with antibodies to both MSA2 (Indochina 1 isolate) and glutathione S-transferase, and it is therefore concluded that this component corresponds to a polypeptide encoded by the SspI fragment of the MSA2 gene fused to GST. The smaller fragments are proteolytic degradation products of the Mᵣ∼70,000 component.

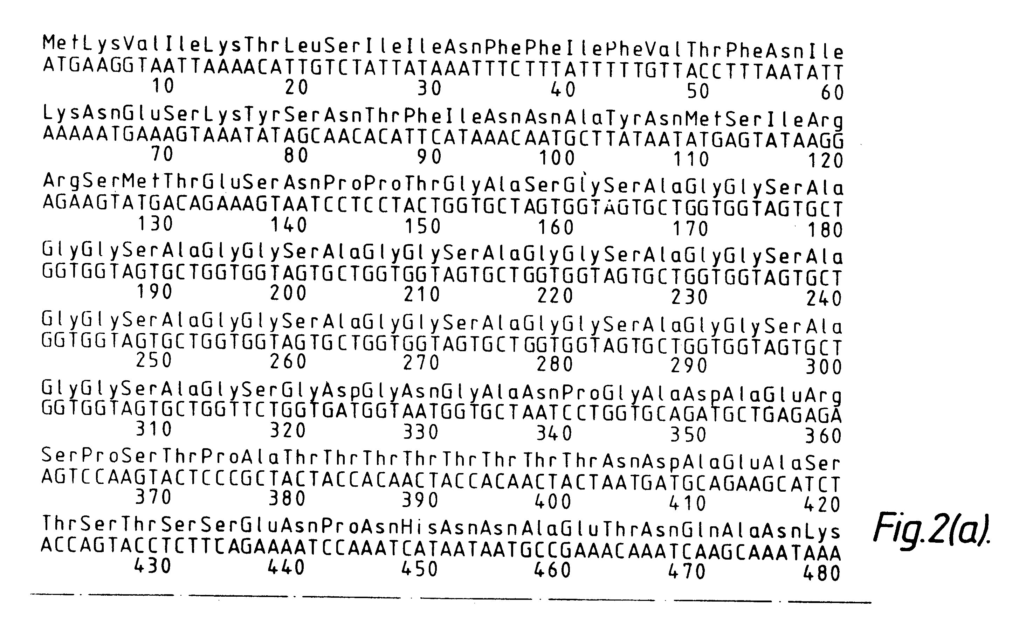

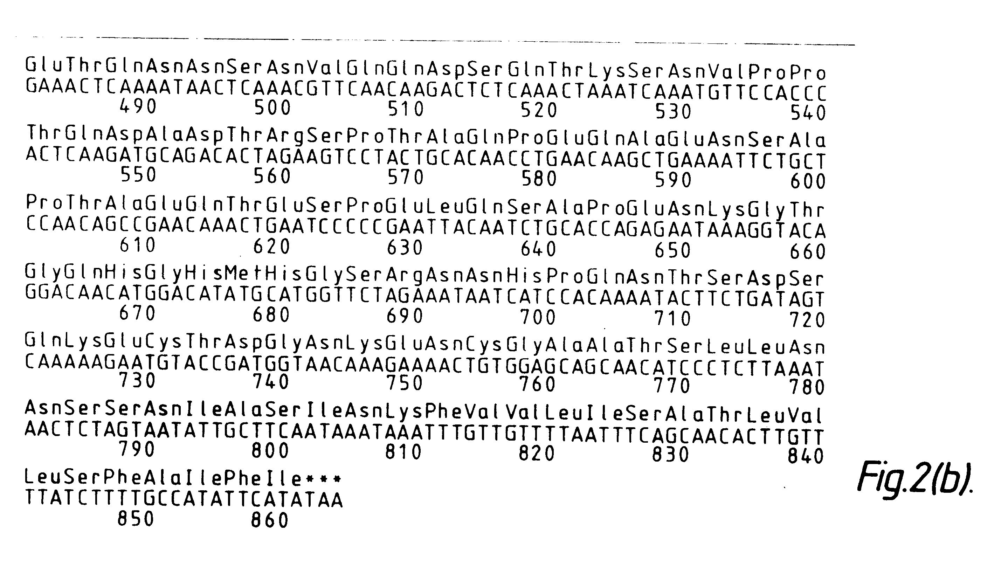

The MSA2 component of this fusion protein (Fig.10 lane 3) has been isolated by cleavage of the affinity purified fusion protein with activated Factor X as described (28) and subsequent separation of the cleaved proteins by reverse phase HPLC. The cleaved purified product (Fig. 10 lane 2) migrates as a single band of Mᵣ 60,000 on SDS PAGE. This molecule reacts with antibodies to MSA2 but not to glutathione-S-transferase. Definitive proof of the identity of this component was obtained by determining the amino acid sequence of the N-terminus of the purified antigen using an Applied Biosystems gas phase protein sequencer, Model 470A, using a 120A PTA analyser according to the manufacturer's instructions. The sequence obtained was as follows:
GIPIKNESKYSNTFINN.
This represents the first three amino acids after the Factor X cleavage site of the vector (28) and 14 consecutive amino acids of the Indochina strain of MSA2 commencing at the isoleucine encoded by nucleotides 58-60 in Fig.2. Amino acid analysis was also performed on the cleaved purified protein. This was determined using a Waters Pico-Tag amino acid analysis system according to the manufacturer's instructions. The composition determined agreed, within experimental error, with the expected composition predicted from the amino acid sequence.

In order to express the Indochina 1 isolate of MSA2 in a "near-native" non-fusion form, the full-length gene was subcloned into pKK223/3 (28c) and expressed in IPTG-induced E.coli cells. Translation was initiated from the MSA2 AUG. Western analysis, following SDS-PAGE showed that the product appeared as two bands of Mᵣ46,000 and 50,000, reacting with sera from PNG malaria immune individuals. This is shown in the autoradiograph in Fig.9a where Lane 1 represents IPTG induced cells, Lane 2 is uninduced cells and Lane 3 is ¹⁴C-labelled size standards. (The two bands are indicated by arrows.)

Using similar techniques, with pGEX-2T(28) as the vector, the SspI fragment of the MSA2 gene from isolate FC27 has also been expressed as a GST fusion protein in E.coli, and isolated by affinity chromatography. The MSA2 component has been isolated after cleavage of the purified fusion protein with thrombin. MSA2 from the FC27 strain of P.falciparum was also expressed as a non-fusion protein in E.coli. The 666 bp Ssp1 fragment of Ag 513(25) was isolated and inserted into the Sma1 site of the vector, pRDB 8 (28a) which contains the 5' and 3' regulatory sequences (including the ATG) of the gene 32 protein of bacteriophage T4 such that the coding sequence of the MSA2 gene was in frame with the ATG of the gene 32 protein gene in pRDB 8. The resulting plasmid was subsequently digested with Cla1 and Dra1. A 922 bp fragment containing the 5' and 3' regions of the gene 32 protein gene flanking the Ssp1 fragment of the MSA2 sequence was isolated and subcloned into the unique Sma1 site of pLK57 (28b). This plasmid was then transformed into E.coli cells for expression. This plasmid-host combination is designated BTA 1893 and the antigen expressed termed Ag1609.

Ag1609 was purified away from E.coli proteins using a combination of ion exchange chromatography and reverse phase HPLC. The molecule migrated as a single band of Mᵣ 50,000 on SDS PAGE (Fig. 10, lane 4), that, on Western blot analysis was immunoreactive with antibodies to MSA2. As with the expressed Indochina 1 MSA2 molecule, N-terminal amino acid sequence analysis was performed and the following sequence was obtained:
XXXXXXXXSXXIKNESKY
where X represents a position to which no amino acid could be confidently ascribed. However, the first 11 amino acids of Ag1609 were expected to have been derived from the cloning vector. The following 7 amino acids match the appropriate 7 amino acids in the FC27 MSA2 sequence described previously(25). Amino acid analysis also confirmed the composition of the recombinant protein as being that expected for the FC27 MSA2.

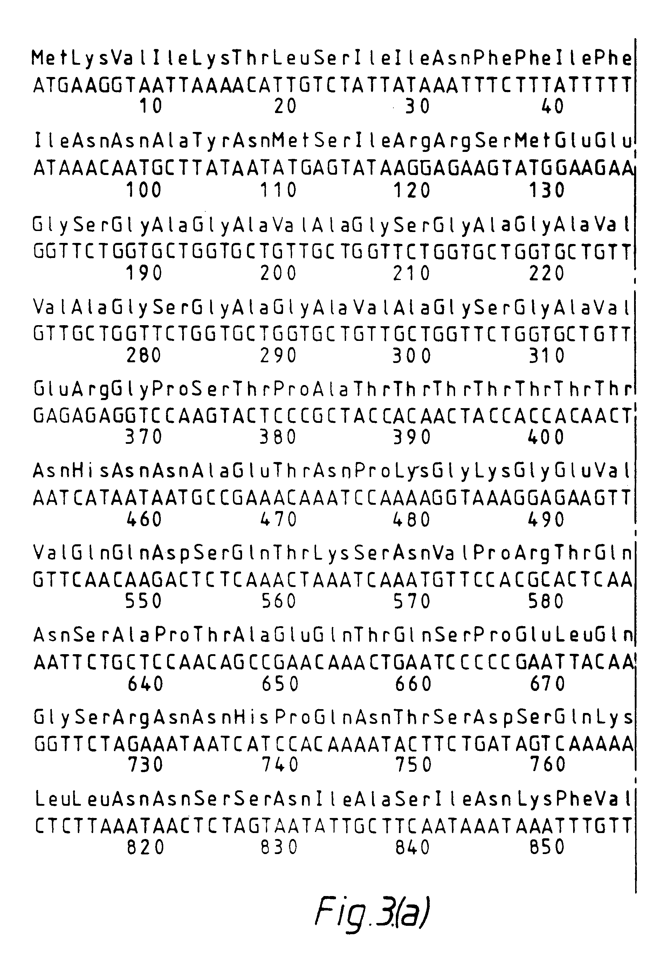

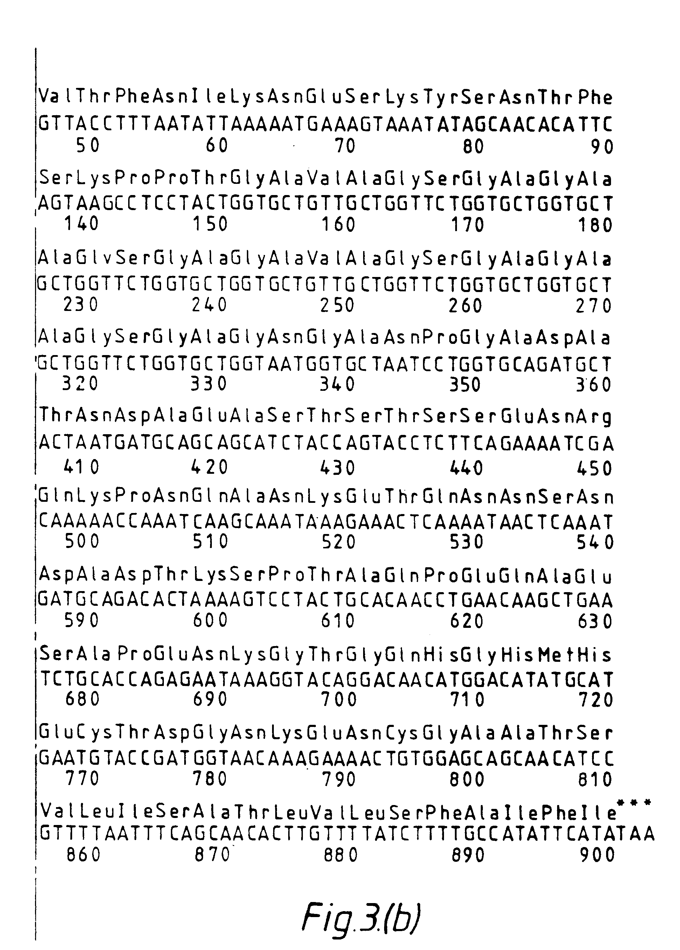

In addition, the conserved C-terminal region of the MSA2 gene from isolate FC27 (nucleotides 661 to 885 in Figure 3 of ref.25) has been expressed in pGEX-2T, and isolated by affinity chromatography. This nucleotide sequence was first amplified from FC27 genomic DNA by PCR using as priming oligonucleotides AAGGATCCATGGCACCAGAGAATAAAGGTAC and AAGGATCCTATGAATATGGCAAAAGATA, with added BamH1 linkers. The amplified DNA included the entire 74 amino acid conserved region of MSA2 except that the N-terminal residue was mutated to replace alanine with methionine. The DNA was gel purified, digested with BamH1, and ligated into the BamH1-cut pGEX-2T vector.

### EXAMPLE 3

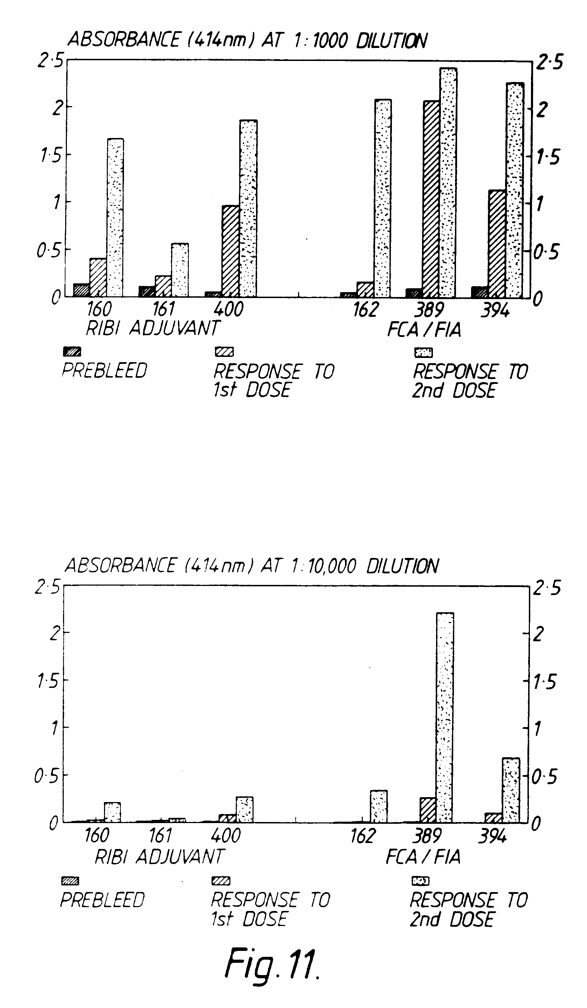

The immunogenicity of MSA2-GST fusion proteins (both FC27 and Indochina 1 forms) and MSA2 of FC27 as a non-fusion protein (Ag1609) has been tested in rabbits. Antibody responses were determined by ELISA using as antigens Ag1609 (which measured the total response of rabbits immunised with the MSA2 of FC27, and the response to the constant regions of rabbits immunised with the MSA2 of Indochina 1) and a synthetic peptide (GGSA)₃ coupled to bovine serum albumin (which measured the repeat-specific response of rabbits immunised with the MSA2 of Indochina 1). Monophosphoryl Lipid A/BCG cell wall skeleton/squalene (RIBI) and Freunds' complete adjuvant (FCA) were used as adjuvants. The results are shown in Figures 11 to 13.

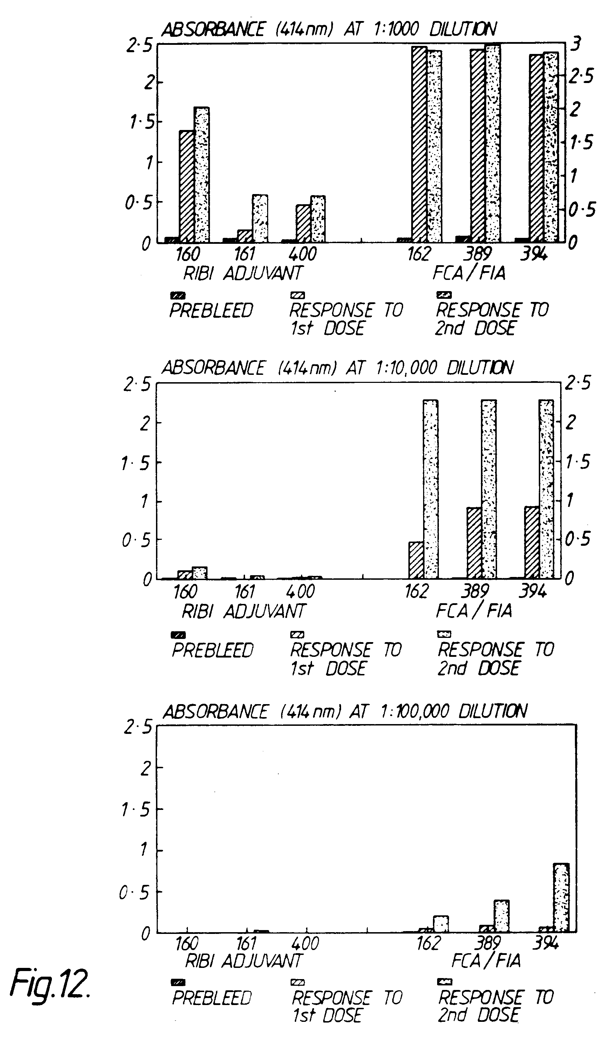

Best responses to the Indochina 1 form of MSA2 (Figures 11 and 12) were obtained when FCA was used as adjuvant; with good responses detected (at 1:10,000 dilution of serum) against Ag1609 and high responses (1:100,000) against the GGSA repeat sequence (Figure 12). With RIBI, the responses measured against Ag1609 were approximately half those obtained with FCA. In contrast, the responses measured against the GGSA peptide were very much lower (by about 30-fold) than those obtained with FCA and were not readily detectable at serum dilutions greater than 1:1000.

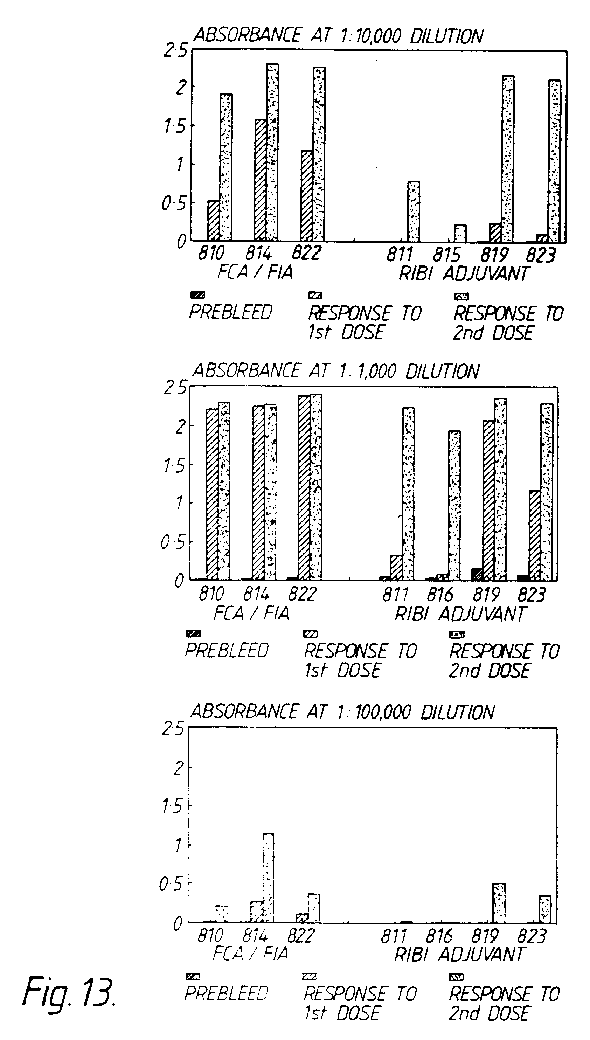

Good responses with both versions of the FC27 form of MSA2 were obtained with FCA, being detected at 1:100,000 dilution of serum (data for Ag1609 in Figure 13). With RIBI, the responses to Ag1609 were lower than those obtained with FCA for some rabbits but were still detectable in all rabbits at 1:10,000 dilution and detectable in 2 of 4 animals at 1:100,000.

### EXAMPLE 4

### A. MATERIALS AND METHODS

### Peptide Synthesis:

41 octapeptides from the N-terminal constant region (E series - E65 to E104) as well as 27 octapeptides from the C-terminal constant region and 10 peptides from the variable region of the Indochina 1 sequence (G series -G1 to G27; G28 to G37) were synthesised using the simultaneous multiple peptide synthesis technique originally described by Houghton(29) employing derivatised (tBOC) amino acids (Omni Biochemicals) on a benzhydrylamine resin (Multiple Peptide Systems).

### Peptide Sequences Employed in the Immunogenicity Trials:

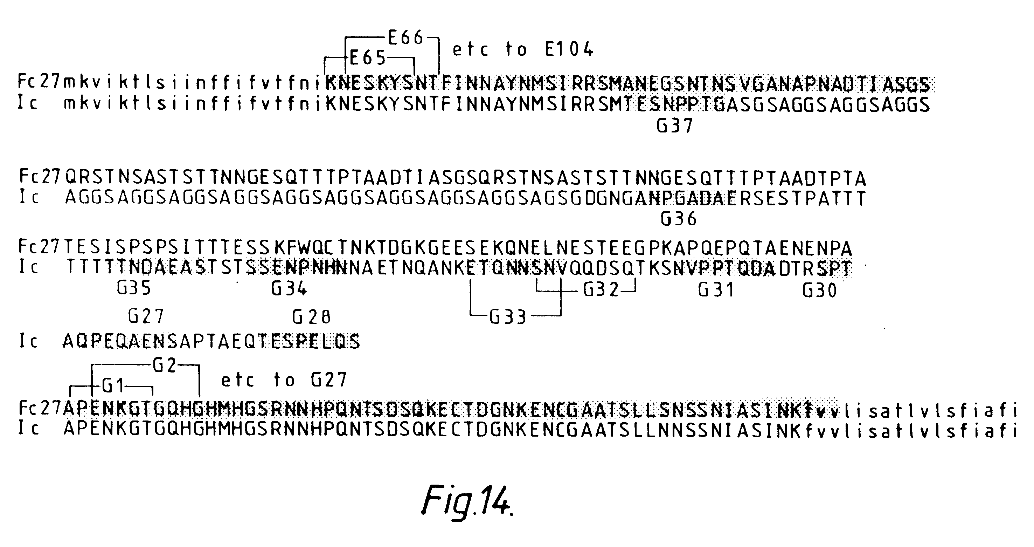

The sequence of MSA2 from the FC27 isolate (FC27) and the Indochina 1 isolate (IC) is shown in Figure 14 using the one letter amino acid code. The lower case letters at the beginning of the sequence show the common N-terminal leader sequence which is cleaved in the mature protein while the lower case letters at the end of the sequence show a portion of the common acylation site sequence which is believed to be cleaved from the mature protein as it is acylated and anchored in the membrane. The deduced amino acid sequence of the mature protein is then represented by the upper case letters. The shaded portions of the sequence show regions from which overlapping or isolated peptides were synthesised and coupled to the carrier protein (DT) for immunogenicity trials. N-terminal peptides (E series) were staggered by one residue at a time as indicated and extended into the variable region of the FC27 sequence. C-terminal peptides (G series) were staggered by two residues at a time and extended into the common cleaved acylation region. Isolated peptides shaded and indicated were also chosen from the variable region of the Indochina sequence. The FC27 (but not the Indochina 1) sequence contains two copies of the STNS epitope (boxed) recognised by inhibitory Mabs 8G10/48 and 9E3/48 (see International Patent Specification No. PCT/AU87/00227).

### Cleavage and Extraction of Peptides:

Cleavage from each of the peptide resin bags (each bag originally contained 100 mg of benzhydrylamine resin) was performed in a multiple HF cleavage manifold (Multiple Peptide systems) using anhydrous HF:anisole:thioanisole: dimethylsulphide (7:0.5:0.5:2) at 0°C for 1 hour. The mixture was then dried under vacuum and the bags washed with cold anhydrous ether, extracted with 10% acetic acid and the crude peptides lyophilised. Yield of crude peptide (60-85% purity) averaged 30 mg.

### Purification of Peptides:

Crude synthetic peptides were dissolved in 5% acetonitrile containing 20 mM dithiothreitol and purified by reverse phase HPLC using an acetonitrile gradient with 0.1% trifluoroacetic acid (TFA) as counterion.

### Coupling of Peptide to Carrier Protein:

Peptides were synthesised with an added N-terminal cysteine to facilitate coupling to the carrier protein and coupled to DT using the heterobifunctional reagent maleimido caproyloxysuccinimide (MCS). High purity DT was obtained from the Commonwealth Serum Laboratories in Melbourne, Australia and derivatized with MCS using the procedure described by Lee et.al.(30). The solution of MCS activated DT protein was added directly to an amount of dry, purified, fully reduced peptide equivalent to 1.4 times the molar equivalent of maleimido groups in the carrier and stirred slowly overnight at room temperature under N₂. The conjugate was then dialysed with several changes against phosphate buffered saline (PBS). The degree of conjugation was determined by titrating the maleimido groups before and after reaction with the peptide using a subtractive Elmans procedure (31). Peptides were also conjugated to bovine serum albumin (BSA) using the same chemistry.

In both cases the extent of coupling was also monitored by observing the increase in apparent molecular weight (SDS/PAGE 8% gels) of the carrier protein-peptide adduct by comparison with the carrier protein itself(32). For both E and G series peptides, apparent molar ratio of peptide to carrier protein ranged from 7-10 for the DT adducts and 8-11 for the BSA adducts.

### Immunization with Peptide-Diphtheria Toxoid Conjugates:

Female Balb/c mice aged 6-7 weeks were pre-bled and then immunized i.p. with the peptide-DT conjugates at 20µg peptide per injection in an emulsion of FCA. One month later, animals received a second injection with incomplete Freund's adjuvant. Two weeks after the final injection animals were bled and the sera inactivated by heating at 56°C for one hour. Inactivated sera was serially diluted in 5% milk powder in phosphate buffered saline (PBS) prior to immunological analysis.

### Antibody Titration:

Wells of flat bottom ELISA plates (Immunolon-Dynatech) were coated overnight with a 5 µg/ml solution of peptide-BSA adducts, blocked with a solution containing 5% skim milk powder in PBS, rinsed in PBS and then incubated either with pre-immunization sera or with sera from mice immunized with the corresponding peptide-DT conjugate serially diluted in 5% milk powder in PBS. After further rinsing in PBS, anti-mouse globulins conjugated to horse-radish peroxidase and the substrate 2,2'azino-di (3-ethylbenzthiazoline sulphonate) (ABTS) were added successively according to the manufacturer's instructions (Amersham, U.K.). The absorbance at 410 nm was measured directly at 0 and 30 minutes using an automated microplate reader. Control plates were coated with sham coupled BSA, i.e. BSA that had been reacted with MCS and blocked with β-mercaptoethanol with no added peptide.

### SDS-PAGE and Immunoblotting:

Parasitized erythrocytes were washed and solubilized with 1% Triton X-100. Protease inhibitors were added (5 µg ml⁻¹ each of pepstatin, leupeptin, chymostatin and antipain). Detergent soluble proteins were resolved on SDS-polyacrylamide gels according to the Laemmli procedure (33). After separation parasite proteins were electrophoretically transferred onto nitrocellulose paper and immunoreacted with mouse antibodies and ¹²⁵I-labelled goat anti-mouse antibody or with rabbit anti-serum and ¹²⁵I-labelled Protein A, following the procedure of Towbin et.al.(34).

### B. RESULTS

### Anti-Peptide Antibodies:

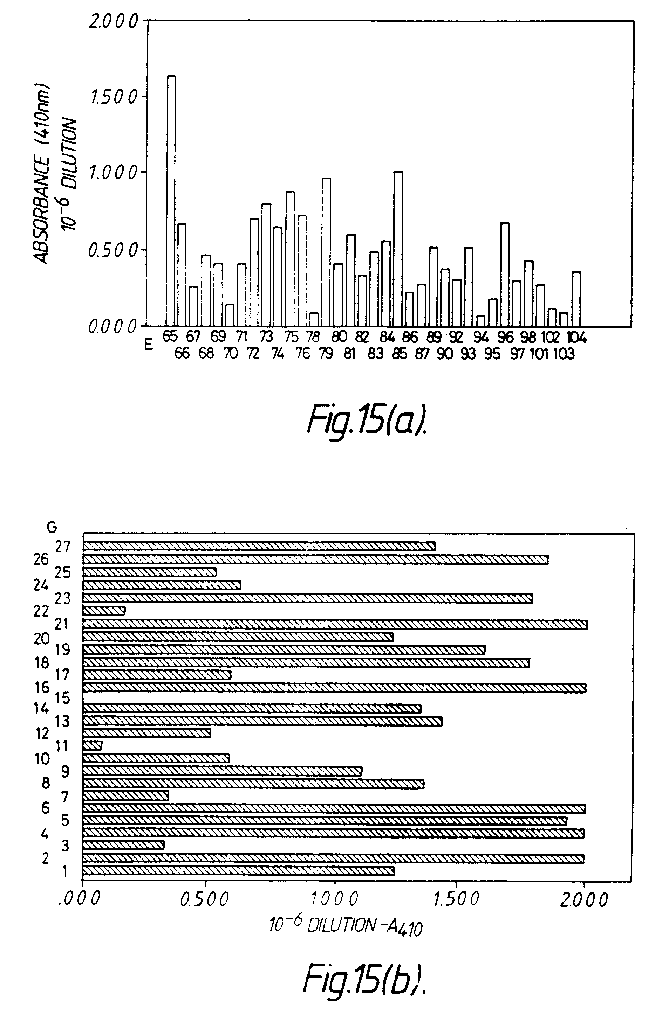

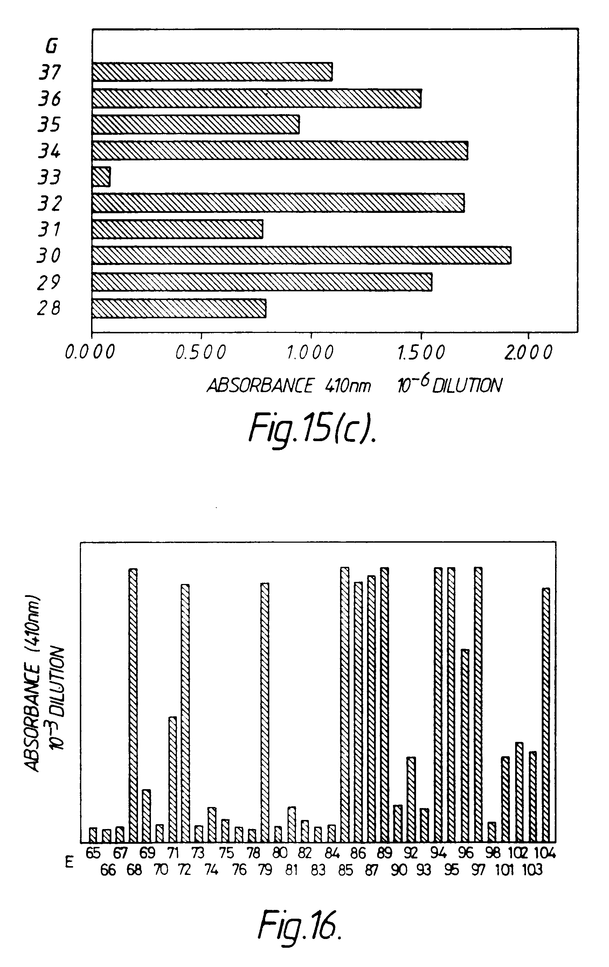

Figure 15(a), (b) and (c) shows the anti-peptide titres for peptides from the conserved N-terminus (E series, Figure 15(a)), C-terminus (G Series, Figure 15(b)) and variable region of the Indochina 1 MSA2 (G series, Figure 15(c)). Results are expressed as the absorbance at 410 nm in the solid phase ELISA assay when the homologous peptide-BSA adduct was used as plate coating antigen and the plates incubated with anti-sera at the indicated dilutions. Most of the peptides are highly immunogenic when presented to Balb/c mice using this protcol. There is however a marked variation in response which is believed to be related to the sequence of the immunising peptide per se since all the sera tested had a high (1/10⁶) titre of anti-DT antibodies when tested against the carrier protein (DT) alone by ELISA. This anti-DT response, although also somewhat variable, did not correlate with the anti-peptide response.

### Anti-Linker Antibodies:

Figure 16 shows the antibody titre (expressed as A₄₁₀ at 10⁻³ sera dilution) when sera from the N-terminal MSA2 peptides (E series) were tested against sham coupled BSA (i.e. BSA-MCS). Clearly some of the peptide-DT adducts elicited quite high anti-linker antibodies. This anti-BSA-MCS response could be inhibited by pre-incubation of the sera with a thiosuccinimido caproylamide (TSC) analogue of the linker region of the peptide-DT conjugate. There is no significant correlation between the levels of anti-peptide and anti-linker response (P = 0.53) for the E series peptides.

### MSA2 Peptides Eliciting an Appropriate Immunofluorescence:

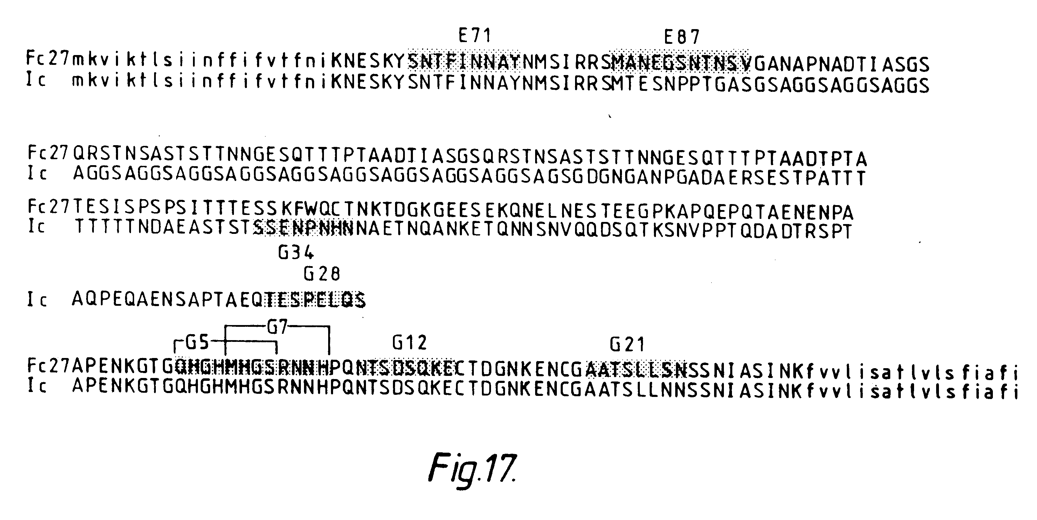

Figure 17 shows the peptide-DT conjugates studied which elicited merozoite surface reacting antibodies when incubated with acetone fixed schizonts. Of the constant region peptides tested, only peptide E71 from the N-terminal portion and peptides G5, G7, G12 and G21 from the C-terminal portion elicited surface reactive antibodies against both FC27 and Indochina 1 parasites. Peptides E87, 88 and 89 elicited IFA positive antibodies against the FC27 parasites only whereas peptides G28 and G34 elicited antibodies against the Indochina 1 parasites only.

### MSA2 Peptides Eliciting an Appropriate Immunoblotting Response:

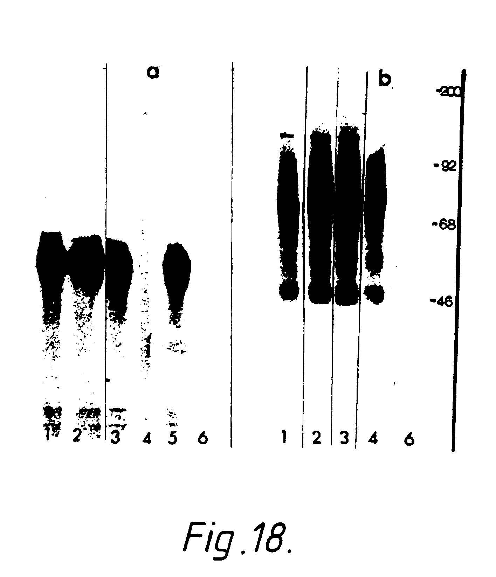

Only peptides E71, G5 and G12 from the MSA2 N- and C-terminal constant regions gave rise to antibodies which reacted with the SDS/PAGE separated Western blotted MSA2 from both FC27 and Indochina 1 parasites (Fig.18(a) and (b)). Peptide E87 elicited antibodies reactive with FC27 only whereas peptide G34 gave rise to antibodies reactive with only the Indochina 1 MSA2. Sera from animals vaccinated with E71, G5 and G12 also gave a positive immunoblotting response against the recombinant full lengh FC27 MSA2 produced in E.coli. Figure 18 shows immunoblotting patterns demonstrated by sera elicited by vaccination with MSA2 peptide-DT conjugates: (a) probed against FC27 extracts separated on SDS/PAGE; (b) probed against Indochina extracts separated on SDS/PAGE.
- Lane 1.: 1/100 dilution of sera from E71 vaccinate;
- Lane 2.: 1/100 dilution of sera from G5 vaccinate;
- Lane 3.: 1/100 dilution of sera from G12 vaccinate;
- Lane 4.: 1/100 dilution of sera from G34 vaccinate;
- Lane 5.: Inhibitory Mab 8G10/48 supernatant; and
- Lane 6.: Pre-bled from E71 vaccinate.

### REFERENCES

1. Mitchell, G.H., Butcher, G.A., Langhorn, J. and Cohen, S. (1977) Clin.Exp.Immunol. 28, 276-279.
2. Mitchell, G.H., Richards, W.H., Butcher, G.A. and Cohen, S. (1977) Lancet 1, 1335-1338.
3. Siddiqui, W.A. (1977), Science 197, 388-389.
4. Freeman, R.R. and Holder, A.A. (1983) J.Exp.Med. 158, 1647-1653.
5. Heidrich, H.G., Strych, W., and Mrema, J.E.K. (1983). Z.Parasitenkd 69, 715-725.
6. Reese, R.T., Motyl, M.R. and Hofer-Warbinek, R. (1981). Am.J.Trop.Med.Hyg. 30, 1168-1178
7. Perrin, L.H., Dayal, R., and Reider, H. (1981) Trans.R.Soc.Trop.Med.Hyg. 75, 163-165.
8. Howard, R.F. and Reese, R.T. (1984) Molec.Biochem.Parasitol. 10, 319-334.
9. Holder, A.A., Lockyer, M.J., Odink, K.G., Sandhu, J.S., Rivercos-Moreno, V., Nicholls, S.C., Hillman, Y., Davey, L.S., Tizard, M.L.V., Schwarz, R.T. and Freeman, R.R. (1985), Nature, 317, 270-273.
10. Holder, A.A. and Freeman, R.R. (1984), J.Exp.Med. 160, 624-629.
11. Lyon, J.A., Geller, R.H., Haynes, J.D., Chinlay, J.D. and Weber, J.L. (1986) Proc.Natl.Acad.Sci. (USA). 83, 2989-2993.
12. Perrin, L.H., Merkli, B., Loche, M., Chizzolini, C., Smart, J. and Richle, R. (1984) J.Exp.Med. 160, 441-451
13. Mackay, M. Goman, M., Bone, N., Hyde, J.E., Scaife, J., Certa, U., Stunnenberg, H. and Binjard, H. (1985) Embo.J. 4, 3823-3829.
14. McBride, J.S., Newbold, C.I., and Anand, R. (1985) J.Exp.Med. 161, 160-180.
15. Weber, J.L., Leininger, W.M. and Lyon, J.A. (1986) Nucl.Acids Res. 14, 3311-3323.
16. Holder, A.A. and Freeman, R.R. (1981) Nature 294, 361-364.
17. Epstein, N., Miller, L.H., Kaushel, D.C., Udeinya, I.J., Rener, J., Howard, R., Asofsky, R., Aikawa, M. and Hess, R.L., (1981), J.Immunol. 127, 212-217.
18. Kilejian, A. (1980), Proc.Natl.Acad.Sci. (USA) 77, 3695-3699.
19. Herrington, D.A. et.al. (1987). Nature 328 : 257-259.
20. Berzins, K. et.al. (1986). Proc.Natl.Acad.Sci (USA) 83 : 1065-1069.
21. Pattarroyo, M.E. et.al. (1980). Nature 332 : 158-161.
22. Richman, S.J. and Reese, R.T. (1988). Proc. Natl. Acad.Sci (USA) 85 : 1662-1666.
23. Saul, A. et.al. In: "Technological Advances in Vaccine Development", Alan Liss Inc., New York (in press).
24. Saiki, R. et.al. (1985). Science 230 : 1350-1354.
25. Smythe, J.A. et.al. (1988). Proc.Natl.Acad.Sci (USA) 85 : 5195-5199.
26. Peterson, M.G. et.al. (1988). ol. Cell Biol. 8 : 2664-2667.
27. Smythe, J., et.al. (1989). In: Vaccines '89, Cold Spring Harbor Laboratory, USA.
28. Smith, D.B. and Johnson, K.S. (1988). Gene 67 : 31-40.
28a. Duvoisin, R.M. et.al. Gene 45 : 193-201.
28b. Botterman, J. and Sabeau, M. (1987). DNA 6 : 583-591.
28c. Brosius, J. and Holy, A. (1984). Proc.Natl. Acad.Sci. USA 81 : 6929.
29. Houghton, R.A. (1985). Proc.Natl.Acad.Sci (USA) 82 : 5131-5135.
30. Lee, A.C.L. et.al. (1980). Mol. Immunol. 17 : 749-756.
31. Sedlack, J. and Lindsay, R.H. (1968). Anal.Biochem. 25: 192-205.
32. Jones, G.L., et.al. J.Immunol.Methods (in press).
33. Laemmli, J.K. (1970). Nature (London) 227 : 680-685.
34. Towbin, H., et.al. (1979). Proc.Natl.Acad.Sci.(USA) 76 : 4350-4354.

## Claims

1. A merozoite surface antigen of Plasmodium falciparum characterised by:
(i) having a relative molecular mass, Mᵣ, (determined by SDS-polyacrylamide gel electrophoresis) in the range of approximately 41,000 to 53,000; and
(ii) comprising one of the multiple allelic forms of MSA2, having conserved N-terminal and C-terminal sequences, other than the allelic form of P.falciparum isolate FC27;
or an antigenic fragment thereof.

2. An antigen according to claim 1, wherein the conserved N-terminal sequence comprises amino acid residues 1 to 43 of Figure 5, and the conserved C-terminal sequence comprises amino acid residues 233 to 307 of Figure 5, or sequences having at least 90% homology thereto.

3. An antigen according to claim 1 or claim 2, having the amino acid sequence set out in Figure 1, Figure 2, Figure 3 or Figure 4, or an antigenic fragment thereof.

4. A vaccine composition comprising an antigen according to any one of claims 1 to 3, or an antigenic fragment thereof, and a pharmaceutically acceptable carrier or diluent.

5. A vaccine composition comprising at least two different merozoite surface antigens of Plasmodium falciparum or two different antigenic fragments thereof, and a pharmaceutically acceptable carrier or diluent, wherein said antigens are characterised by
(i) having a relative molecular mass, Mᵣ, (determined by SDS-polyacrylamide gel electrophoresis) in the range of approximately 41,000 to 53,000; and
(ii) comprising one of the multiple allelic forms of MSA2, having conserved N-terminal and C-terminal sequences.

6. A vaccine composition according to claim 4 or claim 5, wherein said antigenic fragment or fragments comprise or are derived from the conserved N-terminal and/or C-terminal regions.

7. A vaccine composition according to claim 4, further comprising an adjuvant.

8. Use of an antigen as defined in any one of claims 1 to 3 for the preparation of a vaccine composition for actively immunising a host against P. falciparum.

9. A recombinant DNA molecule comprising all or a portion of a nucleotide sequence which encodes a polypeptide according to any one of claims 1 to 3, or an antigenic fragment thereof, or a recombinant cloning vehicle or vector or a host cell comprising a said recombinant DNA molecule.

10. A synthetic polypeptide obtainable by expression of all or a portion of a nucleotide sequence according to claim 9.

11. A vaccine composition comprising a synthetic polypeptide according to claim 10, and a pharmaceutically acceptable carrier or diluent.

12. Use of a synthetic polypeptide obtainable by expression of all or a portion of a nucleotide sequence according to claim 9, for the preparation of a vaccine composition for actively immunising a host against P. falciparum.

13. A synthetic peptide capable of eliciting antibodies which recognise intact MSA2 of P.falciparum, characterised in that it consists of or contains an amino acid sequence selected from the group consisting of:
(i) SNTFINNA;
(ii) QHGHMHGS; and
(iii) NTSDSQKE;
or antigenically active portions thereof.

14. A synthetic peptide capable of eliciting specific antibodies against allelic variants of antigen MSA2 from the FC27, Indochina 1 or other isolates of P. falciparum, characterised in that it consists of or contains an amino acid sequence corresponding to one of the repeat sequences in MSA2, or selected from the group consisting of:
(iv) MANEGSNT;
(v) ANEGSNTN;
(vi) NEGSNTNS; and
(vii) SSENPNHN;
or antigenically active portions thereof.

15. A vaccine composition comprising at least one synthetic peptide according to claim 13 or claim 14, and a pharmaceutically acceptable carrier or diluent.

16. A vaccine composition according to claim 15, wherein said at least one synthetic peptide is coupled or conjugated to a carrier protein.

17. A vaccine composition according to claim 16, wherein said carrier protein is diphtheria toxoid.

18. A vaccine composition according to claim 15 or claim 16 further comprising an adjuvant.

19. Use of a synthetic peptide as defined in claim 13 or claim 14 for the preparation of a vaccine composition for actively immunising a host against P. falciparum.

## Patentansprüche

1. Merozoiten-Oberflächenantigen von Plasmodium falciparum, dadurch **gekennzeichnet**, daß es
(i) eine relative Molekülmasse Mᵣ (bestimmt mittels SDS-Polyacrylamidgelelektrophorese) im Bereich von etwa 41 000 bis 53 000 besitzt; und
(ii)eine der mehrfachen allelen Formen von MSA2 umfaßt, wobei die N-terminalen und C-terminalen Sequenzen konserviert sind, ausgenommen die allele Form des P. falciparum-Isolats FC27;
oder ein antigenes Fragment davon.

2. Antigen nach Anspruch 1, worin die konservierte N-terminale Sequenz die Aminosäurereste 1 bis 43 der Figur 5, und die konservierte C-terminale Sequenz die Aminosäurereste 233 bis 307 der Figur 5 umfaßt, oder Sequenzen mit mindestens 90% Homologie dazu.

3. Antigen nach Anspruch 1 oder 2 mit der Aminosäuresequenz gemäß Figur 1, Figur 2, Figur 3 oder Figur 4, oder ein antigenes Fragment davon.

4. Impfstoffzusammensetzung, umfassend ein Antigen nach einem der Ansprüche 1 bis 3, oder ein antigenes Fragment davon, und einen pharmazeutisch verträglichen Träger oder ein pharmazeutisch verträgliches Verdünnungsmittel.

5. Impfstoffzusammensetzung, umfassend mindestens zwei verschiedene Merozoiten-Oberflächenantigene von Plasmodium falciparum oder zwei verschiedene antigene Fragmente davon und einen pharmazeutisch verträglichen Träger oder ein pharmazeutisch verträgliches Verdünnungsmittel, wobei die Antigene dadurch gekennzeichnet sind, daß sie
(i) eine relative Molekülmasse Mᵣ (bestimmt mittels SDS-Polyacrylamidgelelektrophorese) im Bereich von etwa 41 000 bis 53 000 besitzen; und
(ii)eine der mehrfachen allelen Formen von MSA2 umfassen, wobei die N-terminalen und C-terminalen Sequenzen konserviert sind.

6. Impfstoffzusammensetzung nach Anspruch 4 oder 5, worin das antigene Fragment oder die antigenen Fragmente die konservierten N-terminalen und/oder C-terminalen Regionen umfaßt/umfassen oder davon abgeleitet ist/sind.

7. Impfstoffzusammensetzung nach Anspruch 4, umfassend weiterhin ein Adjuvans.

8. Verwendung eines Antigens nach einem der Ansprüche 1 bis 3 zur Herstellung einer Impfstoffzusammensetzung zur aktiven Immunisierung eines Wirts gegen P. falciparum.

9. Rekombinantes DNA-Molekül, umfassend die Gesamtheit oder einen Teil der Nucleotidsequenz, die ein Polypeptid nach einem der Ansprüche 1 bis 3 oder ein antigenes Fragment davon codiert, oder ein rekombinantes Clonierungsvehikel oder einen rekombinanten Vektor oder eine Wirtszelle, umfassend das rekombinante DNA-Molekül.

10. Synthetisches Polypeptid, erhältlich durch die Expression der Gesamtheit oder eines Teils einer Nucleotidsequenz nach Anspruch 9.

11. Impfstoffzusammensetzung, umfassend ein synthetisches Polypeptid nach Anspruch 10 und einen pharmazeutisch verträglichen Träger oder ein pharmazeutisch verträgliches Verdünnungsmittel.

12. Verwendung eines synthetischen Polypeptids, erhältlich durch Expression der Gesamtheit oder eines Teils einer Nucleotidsequenz nach Anspruch 9 zur Herstellung einer Impfstoffzusammensetzung zur aktiven Immunisierung eines Wirts gegen P. falciparum.

13. Synthetisches Peptid mit der Fähigkeit, Antikörper hervorzurufen, die intaktes MSA2 von P. falciparum erkennen, dadurch **gekennzeichnet**, daß es aus einer Aminosäuresequenz, ausgewählt aus der Gruppe, bestehend aus
(i) SNTFINNA;
(ii) QHGHMHGS; und
(iii) NTSDSQKE;
besteht oder sie enthält, oder antigen wirksame Teile davon.

14. Synthetisches Peptid mit der Fähigkeit, spezifische Antikörper gegen allele Varianten des Antigens MSA2 aus den FC27-, Indochina 1- oder anderen Isolaten von P. falciparum hervorzurufen, dadurch **gekennzeichnet**, daß es aus einer Aminosäuresequenz, entsprechend einer der repetitiven Sequenzen in MSA2 oder ausgewählt aus der Gruppe, bestehend aus
(iv) MANEGSNT;
(v) ANEGSNTN;
(vi) NEGSNTNS; und
(vii)SSENPNHN;
besteht oder sie umfaßt, oder antigen wirksame Teile davon.

15. Impfstoffzusammensetzung, umfassend mindestens ein synthetisches Peptid nach Anspruch 13 oder 14 und einen pharmazeutisch verträglichen Träger oder ein pharmazeutisch verträgliches Verdünnungsmittel.

16. Impfstoffzusammensetzung nach Anspruch 15, worin mindestens ein synthetisches Peptid an ein Trägerprotein gekoppelt oder konjugiert ist.

17. Impfstoffzusammensetzung nach Anspruch 16, worin das Trägerprotein Diphtherietoxoid ist.

18. Impfstoffzusammensetzung nach Anspruch 15 oder 16, umfassend weiterhin ein Adjuvans.

19. Verwendung eines synthetischen Peptids nach Anspruch 13 oder 14 zur Herstellung einer Impfstoffzusammensetzung zur aktiven Immunisierung eines Wirts gegen P. falciparum.

## Revendications

1. Antigène de surface mérozoïte de Plasmodium falciparum caractérisé en ce que :
(i) il possède une masse moléculaire relative, Mᵣ, (déterminé par gel d'électrophorèse de polyacrylamide SDS) dans la gamme d'approximativement 41 000 à 53 000; et
(ii) il comprend l'une des multiples formes alléliques de MSA2, ayant des séquences N-terminales et C-terminales conservées, autre que la forme allélique de l'isolat FC27 de P.falciparum ;
ou un fragment antigénique de celui-ci.

2. Antigène selon la revendication 1, où la séquence N-terminale conservée comprend les résidus amino acides 1 à 43 de la figure 5 et la séquence C-terminale conservée comprend les résidus aminoacides 233 à 307 de la figure 5, ou des séquences ayant au moins 90% d'homologie avec celles-ci.

3. Antigène selon la revendication 1 ou 2, ayant la séquence en acides aminés montrée sur la figure 1, la figure 2, la figure 3 ou la figure 4, ou un fragment antigénique de celui-ci.

4. Composition vaccinale comprenant un antigène selon l'une quelconque des revendications 1 à 3 ou un fragment antigénique de celui-ci, et un véhicule ou un diluant pharmaceutiquement acceptable.

5. Composition vaccinale comprenant au moins deux antigènes de surface mérozoïtes différents de Plasmodium falciparum ou deux fragments antigéniques différents de ceux-ci, et un véhicule ou un diluant pharmaceutiquement acceptable, où lesdits antigènes sont caractérisés en ce que :
(i) ils possèdent une masse moléculaire relative, Mᵣ, (déterminée par gel d'électrophorèse de polyacrylamide SDS) dans la gamme d'approximativement 41 000 à 53 000 ; et
(ii) ils comprennent l'une des multiples formes alléliques de MSA2, ayant des séquences N-terminale et C-terminale conservées.

6. Composition vaccinale selon la revendication 4 ou la revendication 5, où ledit fragment antigénique ou les fragments comprennent ou sont dérivés des régions N-terminale et/ou C-terminale conservées.

7. Composition vaccinale selon la revendication 4 comprenant en outre un adjuvant.

8. Utilisation d'un antigène tel que défini dans l'une quelconque des revendications 1 à 3 pour la préparation d'une composition vaccinale pour immuniser activement un hôte contre P. falciparum.

9. Molécule d'ADN recombinante comprenant tout ou partie d'une séquence de nucléotides qui code pour un polypeptide selon l'une quelconque des revendications 1 à 3, ou un fragment antigénique de celui-ci, ou un véhicule ou un vecteur de clonage ou une cellule hôte recombinant comprenant ladite molécule d'ADN recombinante.

10. Polypeptide synthétique que l'on peut obtenir par l'expression d'une séquence de nucléotides ou d'une portion de celle-ci selon la revendication 9.

11. Composition vaccinaie comprenant un polypeptide synthétique selon la revendication 10 et un véhicule ou un diluant pharmaceutiquement acceptable.

12. Utilisation d'un polypeptide synthétique que l'on peut obtenir par l'expression d'une séquence de nucléotides ou d'une portion de celle-ci, selon la revendication 9, pour la préparation d'une composition vaccinale pour immuniser activement un hôte contre P. falciparum.

13. Peptide synthétique capable de déplacer les anticorps qui reconnaissent le MSA2 intact de P.falciparum,
caractérisé en ce que il consiste en ou contient une séquence d'acides aminés choisis parmi le groupe constitué de :
(i) SNTFINNA ;
(ii) QHGHMHGS ; et
(iii) NTSDSQKE ;
ou des portions actives au plan antigénique de celui-ci.

14. Peptide synthétique capable de déplacer les anticorps spécifiques des variantes alléliques de l'antigène MSA2 de FC27, Indochina 1 ou d'autres isolats de P. falciparum, caractérisé en ce que il consiste en ou contient une séquence d'acides aminés correspondant à l'une des séquences répétées dans MSA2 ou choisies parmi le groupe constitué de :
(iv) MANEGSNT ;
(v) ANEGSNTN ;
(vi) NEGSNTNS ; et
(vii) SSENPNHN ;
ou des portions actives au plan antigénique de celui-ci.

15. Composition vaccinale comprenant au moins un peptide synthétique selon la revendication 13 ou 14 et un véhicule ou un diluant pharmaceutiquement acceptable.

16. Composition vaccinale selon la revendication 15, où au moins un peptide synthétique est couplé ou conjugué à une protéine porteuse.

17. Composition vaccinale selon la revendication 16, où ladite protéine porteuse est la toxine diphtérique.

18. Composition vaccinale selon la revendication 15 ou 16 comprenant en outre un adjuvant.

19. Utilisation d'un peptide synthétique tel que défini dans la revendication 13 ou 14, pour la préparation d'une composition vaccinale pour immuniser activement un hôte contre P. falciparum.
